# EUROPEAN PATENT APPLICATION

(11) **EP 1 854 797 A1**
(43) Date of publication of application: **14.11.2007**
(21) Application number: 06384011.0
(22) Date of filing: 02.05.2006
(51) Int. Cl.: C07D 487/16

(54) **Process for preparing cyameluric chloride**

(71) Applicant: ISDIN, S.A., 08006 Barcelona (ES)
(72) Inventor: Schwarz, Marcus, Leipzigerstr. 29 09596 Freiberg (ES); Buschmann, Helmut H. Lab. del Dr. Esteve, S.A., 08041 Barcelona (ES); Kroke, Edwin, Leipziger Str. 29, 09596 Freiberg (DE); Jiménez Alonso, Oscar, 08006 Barcelona (ES); Holenz, Jörg Lab. del Dr. Esteve, S.A., 08041 Barcelona (ES); Corbera Arjona, Jordi Lab. del Dr. Esteve, S.A., 08041 Barcelona (ES); Payella, David, 08030 Barcelona (ES); Pelejero, Carles, 08030 Barcelona (ES); Trullas, Carles Ramón, 08030 Barcelona (ES)
(74) Representative: Barlocci, Anna

(57) **Abstract**

The present invention concerns an improved process for obtaining larger quantities (several 10 grams up to kilograms) of pure cyameluric chloride (I) by chlorination of cyameluric acid (C₆N₇(OH)₃) or its alkaline salts followed by fractioned vacuum sublimation.

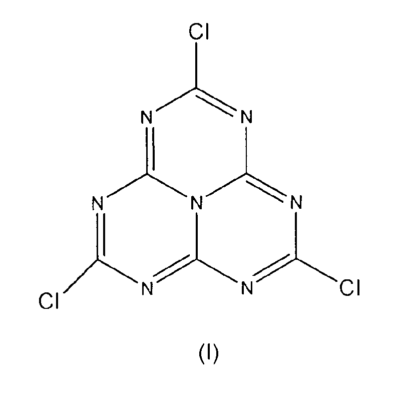

## Description

### FIELD OF THE INVENTION

The present invention concerns an improved process for obtaining larger quantities (several 10 grams up to kilograms) of pure cyameluric chloride (I) by chlorination of cyameluric acid (C₆N₇(OH)₃) or its alkaline salts followed by fractioned vacuum sublimation. 2,5,8-trichloro-1,3,4,6,7,9,9b-heptaazaphenalene of the formula (I) 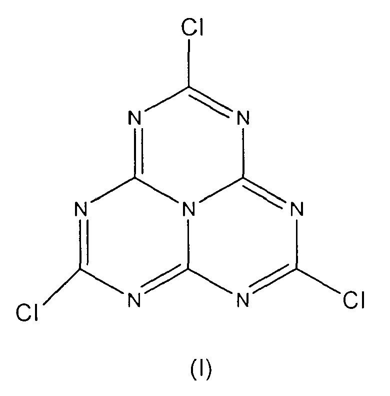 is also known as 2,5,8-trichlorotri-s-triazine and designated as cyameluric chloride in this invention.

### BACKGROUND OF THE INVENTION

Aromatic nitrogen heterocycles have a wide variety of uses in coordination chemistry and optical science. In this sense, s-triazine is widely used in synthetic chemistry, coordination chemistry and optical and magnetic studies, also is playing a key role in molecular routes to carbon nitride (CNₓ) materials. On the other hand having as final objective to incorporate larger C-N fragments in precursors of this aromatic nitrogen heterocycles, one of the most studied ones has been the 1,3,4,6,7,9,9b-heptaazaphenalene ring system (also known as tri-s-triazine or s-heptazine) (see Miller,D.R.; J. Am. Chem. Soc.; (2004); 126; 5372-5373).

In this way, the "azaaromatic chlorides" which are defined as aromatic compounds containing many C=N-Cl subunits, represents a remarkable class of intermediates which display extensive networks of intermolecular nitrogen-chloride donor-acceptor interactions in the solid state as has been described in the literature (see Xu, K.; J. Am. Chem. Soc.; (1994); 116; 105).

Cyameluric chloride is a valuable intermediate for the synthesis of different derivatives, such as gelling agents for aliphatic hydrocarbons (see US 3,089,875), fluorine-containing oxidants which can be used as additives for rocket propellants (see US 3,202,659), stabilizers for photographic emulsions (see US 2,704,716 and US 2,801,172), dyes (see DE 1,102,321), graphitic C₃N₄ structures as a suitable precursors of superhard C₃N₄ phases (see Kroke,E.; New J. Chem.; (2002); 26; 508-512). It also is an intermediate for the preparation of several variously usable heptazine derivatives that has been pointed out elsewhere (see Pauling,L.; Proc. Nat. Acad. Sci. US; (1937); 23; 615-620; DE 2,106,675; US 4,205,167; furthermore Finkel'shtein,A.L.; Russ. Chem. Rev.; (1964); 33(7); 400-405).

At present two fundamentally different procedures are known for obtaining the cyameluric chloride of formula (I). On one hand compound (I) is obtained by reaction of C₆N₇O₃(R)₃, cyameluric acid (when R=H) or an alkaline cyamelurate (when R=Na, K, etc) with phosphorous pentachloride in a closed system (see Redemann,C.E.; J. Am. Chem. Soc.; (1940); 62; 842-846). In this original procedure, done in solid state without any solvent or liquid phase in a sealed tube at 230 °C, a pressure develops due to formation of volatile POCl₃ and, in order to avoid bursting of the ampoule, the reaction had therefore occasionally to be interrupted and the positive pressure to be discharged.

In a later work of Schroeder and Kober (see Schroeder,H.; J. Org. Chem.; (1962); 27; 4264) it is reported that the reaction using a sealed bomb tube did not have to be interrupted and that after finishing and upon opening of the tube no positive pressure was detectable. Attempts to avoid the use of a sealed system by replacing the PCl₅ with phenylphosphorous tetrachloride (C₆H₄PCl₄) were not successful.

According to Neeff (see DE 1,102,321) it is possible to perform the chlorination in an open system using boiling o-dichlorobenzene as a reaction medium or solvent. However, aromatic chlorinating solvents are frequently carcinogens. They are difficult to be completely removed from the product due to their relatively low volatility and high affinity to the aromatic π-system of the heptazine. Such contamination is particularly problematic, if the final product synthetized from the cyameluric chloride is to be used in a human environment.

The separation of side products of the chlorination (phosphoryl chloride POCl₃, alkali chloride and possibly phosphates) was accomplished by both procedures, Redemanns and Schroeder, via washing with water followed by vacuum filtration. This is disadvantageous, since cyameluric chloride is very sensitive to hydrolysis. Even when ice water at 0°C is used (see Schroeder procedure) a large part of the product is decomposed to form cyameluric acid.

Own attempts of the inventors indicated that the fine particle size of the cyameluric chloride caused long filtration times. This implies - in combination with the residual moisture of the filter cake - a problem, to which also by use of a filter press as well as immediate evacuation of the filter cake in a desiccator over phosphorous pentoxide could not be solved satisfactorily.

Further attempts to purify the product by sublimation under atmospheric pressure and with high vacuum were not successful according to Redemann reference.

Experiments of the inventors with a commercial sublimation set-up consisting of a vacuum recipient and a water-cooled separating surface, confirmed these findings.

Pure cyameluric chloride can be obtained by a procedure described by Gremmelmaier (see US 4,205,167). In this patent, chlorine cyanide CI-CN or evaporated cyanuric chloride is transferred to a cyameluric chloride under addition of atmospheric oxygen at an activated charcoal catalyst at 400 °C. The cyameluric chloride which is formed is obtained as a mixture with remaining starting material cyanuric chloride. The product is collected in a flask which is heated to 200 °C, while the cyanuric chloride condenses in another flask at 20 °C. This procedure is characterized by the following disadvantages: relative low yield (31.7 mass percentatge of the cyanuric chloride used as starting material), relatively complicated set-up because mass flow controllers are required for controlling the amount of starting material and air, difficult adjustment of the evaporation rate of solid cyanuric chloride and a multiple zone furnace and/or several furnaces with separate regulation, arranged into a row and connected with joints which have to be heated to >200 °C are necessary in order to prevent condensation of the product which might cause problems with the tightness of the glass joint between the reactor and the receptacles, specially the temperature stability of the used sealing material.

### DISCLOSURE OF THE INVENTION

The present invention includes several improvements on conditioning of the raw material, the chlorination and the purification of the final product. It was found that a commercially available technical product, the polymer melon (designated as melon powder in this invention and identified with CAS RN: 32518-77-7), can be used as a heptazine source.

In one aspect of the invention the process for obtaining cyameluric chloride (I) from cyameluric acid or an alkaline salt of it is characterized in that cyameluric acid or an alkaline salt of it is obtained from purified melon powder.

It turned out to be favourable to remove production-determined existing melamine as well as other molecular cyanamide derivatives, which are present as contaminants in the starting material (melon powder).

Different methods were successfully applied: calcinations at 560 °C under an inert atmosphere causes condensation reactions of the low molecular weight contaminants forming heptazine units, removal of volatile impurities by evaporation/sublimation, and extraction or washing the polymeric starting material thoroughly with water.

In one preferred embodiment the method applied for removing melamine as well as other molecular cyanamide derivatives from the starting material is by pyrolysis that is carried out applying the following temperature scheme:
a. heating from room temperature to between 100 °C and 200°C, preferably from room temperature to 120 °C in 60 minuts, preferably in 30 minutes
b. maintain between 100 °C and 200°C, preferably maintain 120 °C during at least 2 hours, preferably during 4 hours
c. heating from between 100 °C and 200°C to between 400 °C and 800°C, preferable from 120 °C to 560 °C in between 1 and 3 hours, preferably in 2 hours
d. maintain between 400 °C and 800°C, preferably maintain 560 °C during at least 1 hour, preferably during 2 hours followed by cooling down.

In another embodiment the method applied for removing melamine as well as other molecular cyanamide derivatives from the starting material is via Soxhlet-extraction with water during at least 24 hours, preferably during 48 hours and posterior evaporation of the resulting aqueous solution by known means as rotation evaporator.

Finally in another embodiment the method applied for removing melamine as well as other molecular cyanamide derivatives from the starting material is via extraction with water, preferably at temperatures up to 85 °C.

In particular melamine otherwise causes problems during the following process steps as impurities in the form of cyanurates such as (H₃₋ₓC₃N₃O₃)^{x} or cyanuric chloride. The latter leads, due to its higher vapour pressure to a poorer vacuum during the double sublimation used to obtain the final product, and thereby to significantly decreased yields per time unit.

The chlorination of C₆N₇O₃(R)₃ (with R as defined above) is preferably performed with PCl₅, preferably at atmospheric pressure and using preferably phosphoryl chloride POCl₃ as reaction medium or solvent. The POCl₃ is at the same time side product of the reaction between the C₆N₇O₃(R)₃ and PCl₅.

It can be recovered by distillation and it is convertible into harmless and environmentally compatible products by hydrolysis, which is contrary to alternative solvents like high-boiling and chlorine containing aromatic solvents.

In this invention the term "atmospheric pressure" means pressures between 0.5 atm and 1.5 atm, preferably 1 atm.

Also in this invention the term "alkaline salt" means that R can be selected from sodium, potassium etc, preferably with complete substitution (three atoms) but includes any mixture wherein this substitution could not be complete.

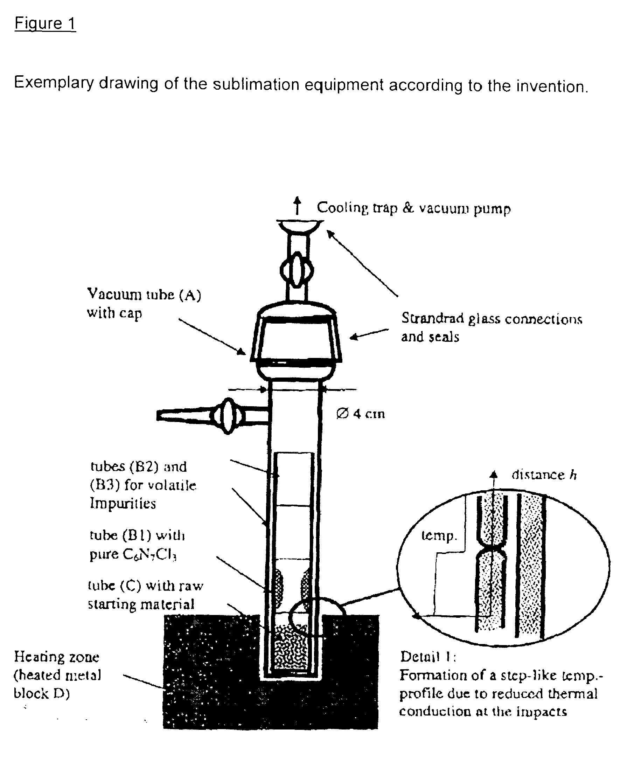

It was shown that cyameluric chloride is to be preferably sublimated in a sublimation apparatus with tubular geometry, as illustrated in figure 1, since in this case substantially higher yields are obtained and, at the same time, substantially pure products result.

Very pure cyameluric chloride (I) is obtained by fractioned vacuum sublimation in a tube-shaped, double walled (glass) set-up (figure 1). This apparatus consists of an outside vacuum tube (A) in which the inner (smaller) tubes [(B1), (B2) and (B3)] are concentrically located.

By the decreased heat flow at the axial contact surfaces of the tubes (see enlarged detail in figure 1) and by the radial isolation in relation to the environment due to the vacuum, a step-like temperature profile is formed. In consequence, the temperature difference within a single inner pipe is small, but significantly bigger between successive pieces (see enlarged detail in figure 1).

Therefore, the purified product deposits in the inner pipe (B1) directly above the heating zone, while the side products with high volatility deposit on (B2) and (B3) or are collected in one of the cooling traps. The first inner piece of pipe (B1) with the purified product may -after the sublimation is finished- mechanically be separated from the other pipes as well as the lower part of the glass set-up (C) which contains low or non-volatile impurities and/or the raw material. The pure cyameluric chloride (I) can easily be collected without any contamination.

If a sufficient pumping power is available, as many sublimation tubes as desired can be operated in parallel at the same time.

The following examples will further illustrate the invention; they should not be interpreted as limiting the scope of the invention.

### Examples

### 1. Conditioning of the raw melon powder.

### 1.1. Conditioning by pyrolysis

100 g pelletized melon was filed in a beaker and stored for 48 hours in a drying oven at 120 °C. The weight of the obtained product was 97.4 g (that implies 2.8 % of water in the starting material). The dried starting material was transferred to a quartz glass tube and evacuated three times to ~ 10⁻¹ mbar and flushed with dry argon. This pre-treated raw melon was then heated in the quartz glass pipe under an inert gas flow of nitrogen of approximately 10 I/h applying the following temperature program:
a. heating from 20 °C to 120 °C in 30 minutes
b. maintain 120 °C during 4 hours
c. heating from 120 °C to 560 °C in 2 hours
d. maintain 560 °C during 2 hours
Cooling by switching the heating off.

In the colder zone of the quartz tube 4.2 g of a heterogeneous sublimate (with white and yellow colour) has separated. The white material was identified as melamine by infrared spectroscopy. The weight of the obtained calcined melon was 86.8 g (yield 89 %), related to the pre-dried melon. The observed weight difference to the 93.2 g (m_{(raw melon)} - m_{(sublimated material)}) is due to the formation of ammonia (NH₃) during the heat treatment (approximately 6.4 g were formed). The X-ray powder diffraction pattern of the heat treated melon resembles the material reported in Komatsu (see Komatsu,T.; Macromol. Chem. Phys.; (2001); 202; 19-25) which was designated as "orthorhombic melon". The overall yield related to the assigned melon results thereby too 86.8 %.

### 1.2. Conditioning via Soxhlet-extraction with water.

Pelletized melon was milled into a fine powder. 64.4 g of this fine powder were placed in a Soxhlet-extraction set-up and extracted for 48 hours with distilled water. The obtained aqueous solution was evaporated by means of a rotation evaporator, resulting in 5.1 g of a white powder, which was analyzed to be melamine. The weigh of the purified melon which remained in the Soxhlet-extractor was determined after drying to be 57.5 g. The overall yield related to the assigned, non pre-dried melon results thereby to 89 %.

### 1.3. Conditioning via extraction with water.

1 kg of pelletized melon was milled to a fine powder, dispersed in 2 litres of distilled water and refluxed in a 4-litre flask. The hot mixture was filtered using a filter nut and a vacuum pump. This procedure was repeated a second time with fresh distilled water. The overall yield related to the assigned, non pre-dried melon results to 925 g (yield 92%). Therefore, it is concluded that 2% of melamine still remains in the purified product.

### 2. Preparation of potassium cvamelurate_{.} K₃[C₆N₇O₃]

The synthesis and drying of the potassium cyamelurate from the purified and conditioned melon was performed in accordance with procedures known in the state of the art (see Redemann,C.E.; J. Am. Chem. Soc.; (1939); 61; 3420-3424).

### 2.1. Preparation of cyameluric acid, H₃[C₆N₇O₃]

The synthesis of the cyameluric acid was performed in accordance with standard procedures known in the state of the art as it is by acidifying a solution of its alkaline salt, previously obtained as indicated above. The acid, that has a very slow solubility in water, precipitates from the solution and can be separated by filtration.

### 3. Chlorination reaction

### 3.1. Chlorination of potassium cyamelurate with POCl₃ as a reaction media

In a 1-litre two-necked flask equipped with reflux condenser and side stop-cock were placed 500 ml of POCl₃. Under flowing inert gas and stirring a powdered mixture of 93.2 g (0.44 mmol) of PCl₅ and 50 g (0.14 mmol) of dried potassium cyamelurate were added using a powder funnel. The mixture was then refluxed for 4 h, whereby a bright yellow colour developed gradually.
The POCl₃ and the excessive PCl₅ were removed by distillation using a short glass tube connection instead of a distillation bridge (temperature of the oil bath was approximately 165 °C). The yellow residue in the flask was transferred into a glove-box with gas regeneration (≤0.5 ppm oxygen; ≤0.2 ppm water). After sublimation purified cyameluric chloride was obtained as described below.

### 3.2. Chlorination of cyameluric acid with POCl₃ as a reaction media

In a 1-litre two-necked flask equipped with reflux condenser and side stop-cock were placed 500 ml of POCl₃. Under flowing inert gas and stirring a powdered mixture of 100.0 g (0.472 mmol) of PCl₅ and 31.0 g (0.14 mmol) of dried cyameluric acid were added using a powder funnel. The mixture was then refluxed for 6 h, whereby a vigorous reaction was observed and a yellow colour developed gradually.
The POCl₃ and the excessive PCl₅ were removed by distillation using a simple glass tube connection (temperature of the oil bath was approximately 160 °C). The yellow residue in the flask was transferred into a glove-box with gas regeneration (≤0.5 ppm oxygen; ≤0.2 ppm water). After sublimation (see below) less than 2 g of cyameluric chloride was obtained as described below.

### 4. Purification of cyameluric chloride by vacuum fractioned sublimation

20 g of raw cyameluric chloride obtained as described under section 3.1 were transferred in a glove-box to a glass tube labelled C in figure 1. This tube was placed into the (outer) sublimation tube shown in figure 1. The other glass tube pieces (B1), (B2) and (B3) were then also introduced into the (outer) sublimation tube.

The purification of the raw cyameluric chloride was performed using four sublimation tubes of the type shown in figure 1 in parallel, i.e. at the same time. The latter four sublimation tubes were connected to a vacuum system via a manifold.

The vacuum system consisted of a two-stage rotary oil pump combined with an oil diffusion pump (maximum end pressure ~ 10⁻⁶ mbar). The pressure during the sublimation was ~10⁻³ mbar. The pumps were protected against corrosive chemicals by two double-walled cooling traps and cooled with liquid nitrogen.

First, the argon filled sublimation tubes were carefully evacuated at room temperature. When the vacuum was <10⁻² mbar, the metal block (part (D) in figure 1) was heated to 50 °C. After 30 min. the temperature was increased to 150 °C and hold at this level fro 45 min. This procedure served for removing volatile contaminants such as POCl₃, PCl₅ etc.

Afterwards, the temperature was increased to 245 °C and hold for 6 h 30 min. During this period, the vacuum reached from 4x10⁻³ to 6x10⁻³ mbar. The evacuated sublimation tubes were cooled to room temperature and transferred to a glove-box again. Purified cyameluric chloride (I) was obtained as a bright yellow layer with a thickness of up to 1 cm, which firmly sticks to the glass walls of tube B1.

The yield of one sublimation cycle was up to 20 g (≈ 25% relative to raw melon powder from point 1.1).

## Claims

1. A process for obtaining cyameluric chloride (I) from cyameluric acid or an alkaline salt of it **characterized in that** cyameluric acid or an alkaline salt of it is obtained from purified melon powder.

2. The process according with claim 1 **characterized in that** melon powder is purified by pyrolisis.

3. The process according to claim 1 and 2 **characterized in that** the pyrolysis is carried out applying the following temperature scheme:
a. heating from room temperature to between 100 °C and 200°C, preferably from room temperature to 120 °C in 60 minuts, preferably in 30 minutes
b. maintain between 100 °C and 200°C, preferably maintain 120 °C during at least 2 hours, preferably during 4 hours
c. heating from between 100 °C and 200°C to between 400 °C and 800°C, preferable from 120 °C to 560 °C in between 1 and 3 hours, preferably in 2 hours
d. maintain between 400 °C and 800°C, preferably maintain 560 °C during at least 1 hour, preferably during 2 hours followed by cooling down.

4. The process according to claim 3 **characterized in that** after pyrolysis temperature is cooled down below 50 °C, preferably until room temperature.

5. The process according to claim 1 **characterized in that** the melon powder is purified by extraction with water, preferably at temperatures up to 80 °C.

6. The process according to claim 1 **characterized in that** the melon powder is purified via Soxhlet-extraction with water at least during 24 hours, preferably during 48 hours.

7. The process according to claims 1 to 6 **characterized in that** the main impurity eliminated is melamine.

8. A process for obtaining cyameluric chloride (I) 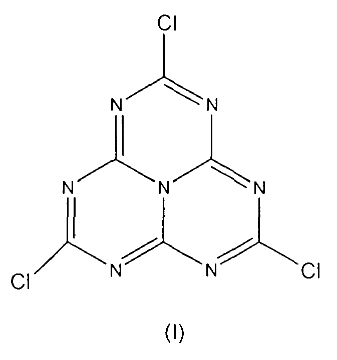 **characterized by** chlorination of cyameluric acid or an alkaline salt of it with phosphorous pentachloride (PCl₅) in phosphorous oxychloride (POCl₃).

9. The process according to claim 8 **characterized in that** the alkaline salt is preferably potassium cyamelurate.

10. The process according to claims 8 and 9 **characterized in that** the chlorination reaction is performed under reflux conditions.

11. The process according to claims 8 to 10 **characterized in that** the chlorination reaction is performed at atmospheric pressure.

12. The process according to claims 8 to 11 wherein the cyameluric acid or an alkaline salt of it is obtained from purified melon powder according to claims 1 to 7.

13. A process for obtaining cyameluric chloride **characterized in that** the cyameluric chloride (I) is purified by vacuum fractioned sublimation through the apparatus described in figure 1.

14. The apparatus according with claim 13 **characterized in that** the outside vacuum tube (A) in which the inner (smaller) tubes [(B1), (B2) and (B3)] are concentrically located.

15. The apparatus according with claims 13 and 14 **characterized in that** by the decreased heat flow at the axial contact surfaces of the tubes (see enlarged detail in figure 1) and by the radial isolation in relation to the environment due to the vacuum, a step-like temperature profile is formed.

16. The apparatus according with claims 13 to 15 **characterized in that** the temperature difference within a single inner pipe is small, but significantly bigger between successive pieces (see enlarged detail in figure 1).

17. The apparatus according with claims 13 to 16 **characterized in that** the purified product deposits in the inner pipe (B1) directly above the heating zone, while the side products with high volatility deposit on (B2) and (B3) or are collected in one of the cooling traps.

18. The apparatus according with claims 13 to 17 **characterized in that** first inner piece of pipe (B1) with the purified product may, after the sublimation is finished, mechanically be separated from the other pipes.

19. The apparatus according with claims 13 to 18 **characterized in that** the lower part of the glass set-up (C) which contains low or non-volatile impurities and/or the raw material may, after the sublimation is finished, mechanically be separated from the other pipes.

20. The apparatus according with claims 13 to 19 **characterized in that** depending on the pumping power available, as many sublimation tubes as desired can be operated in parallel at the same time.

21. The process according with claim 13 wherein the vacuum fractioned sublimation steps are carried out with vacuum values from <10⁻² mbar to 6x10⁻³ mbar and temperatures from room temperature to 250 °C.

22. A process according with claims 8 to 12 for obtaining cyameluric chloride (I) by chlorination of cyameluric acid or an alkaline salt of it, the cyameluric chloride (I) obtained is purified by vacuum fractioned sublimation **characterized in that** the vacuum fractioned sublimation is carried out through an apparatus according with claims 13 to 21.

23. A process according with claims 8 to 12 for obtaining cyameluric chloride (I) by chlorination of cyameluric acid or an alkaline salt of it, **characterized in that** cyameluric acid or an alkaline salt of it is obtained from purified melon powder according with claims 1 to 7 and cyameluric chloride (I) is purified by vacuum fractioned sublimation carried out through an apparatus according with claims 13 to 21.
